# EUROPEAN PATENT SPECIFICATION

(11) **EP 1 505 058 B1**
(45) Date of publication and mention of the grant of the patent: **10.12.2014**
(21) Application number: 03725930.6
(22) Date of filing: 21.04.2003
(51) Int. Cl.: C07C 211/63, C07C 209/12

(54) **METHOD FOR PRODUCING ALKYLDIMETHYLBENZYLAMMONIUM CHLORIDES**
VERFAHREN ZUR HERSTELLUNG VON ALKYLDIMETHYLBENZYLAMMONIUMCHLORIDEN
PROCEDE DE FABRICATION DE CHLORURES D'ALKYLDIMETHYL-BENZILAMMONIUM

(30) Priority: 24.04.2002 RU 2002110966
(43) Date of publication of application: 09.02.2005
(73) Proprietor: Nedd Marketing SA, Moscow, 129226 (RU)
(72) Inventor: ZOTOV, Vyacheslav Ivanovich, Moscow, 109377 (RU); MANKOVICH, Leonid, 26337 Kiryat-Motskin (IL)
(74) Representative: Gulde & Partner
(86) International application number: PCT/RU2003/000183
(87) International publication number: WO 2003/091197

(56) References cited:
- JP-A- 6 157 432
- JP-A- 8 268 982
- RU-C1- 2 123 491
- US-A- 3 385 893
- US-A- 4 442 306
- US-A- 5 545 749
- E. AVRAM: REVUE ROUMAINE DE CHIMIE, vol. 46, no. 1, 2001, pages 49-53, XP008046703
- J.M. KAMINSKI ET AL: JOURNAL OF THE AMERICAN OIL CHEMISTS SOCIETY, vol. 54, no. 11, 1977, pages 516-520, XP008046707
- DATABASE CA [Online] ZHANG YING ET AL.: 'Synthesis and determination of benzyldodecyldimethyl quaternary ammonium salts cationic surfactant', XP002992593 Database accession no. 132:167994 & ZENGKAH, PROCEEDINGS OF CHINA STH SYMPOSIUM ON INDUSTRIAL SURFACTANT TECH-ECONOMIC APPLICATION DEVELOPMENT 1999, pages 116 - 118
- DATABASE CA [Online] AVRAM ECATERINA: 'Quaternization reaction of some N,N-dimethylalkylamines with benzyl chloride', XP002992594 Database accession no. 137:384627 & REVUE ROUMAINE DE CHIMIE vol. 46, no. 1, 2001, pages 49 - 53

## Description

### Field of the Invention

The inventive method relates to a method for producing disinfectant mixtures and quaternary ammonium, namely dodecyl dimethylbenzyl ammonium chloride and tetradecyl dimethylbenzyl ammonium chloride, compositions, and is applicable in the production of household chemical goods.

### Background of the Invention.

There is a known method for producing alkyldimethyl benzyl ammonium chlorides [1] or benzalconium chloride with a general chemical formula of R(CH₃)₂NCH₂C₆H₅Cl by interaction of tertiary amines with benzyl chloride:

R(CH₃)₂N + C₆H₅CH₂Cl → R(CH₃)₂NCH₂C₆H₅Cl (1),

where R is the aliphatic hydrocarbon radicals (alkyl radicals) containing between 8 and 25 carbon atoms.

The basic constituents of a tertiary amine mixture were amines with the alkyl radicals containing 12 and 14 carbon atoms; however, said mixture contained a large quantity of tertiary amines with the alkyl radicals containing more than 14 carbon atoms, which increased the toxicity of the end product, alkyldimethyl benzyl ammonium chlorides. Furthermore, a wide range of tertiary amines with different molecular masses conditions proceeding of both principal chemical reactions of benzyl chloride with tertiary amines at different rates, and of various possible side reactions on account of likely combinations of interaction between different substances. This eventually determines existence of a large quantity of diverse impurities in the end product and, respectively, unpredictable and undesirable changes in its useful functional properties.

It should be noted that according to sources [2] and [3], the toxicity of the end product increases on account of presence therein of unreacted low-molecular and high-molecular tertiary amines.

There is a known method for producing alkyldimethyl benzyl ammonium chloride, namely dimethyl cetyl benzyl ammonium chloride (C₁₆), by interaction of refined tertiary amine, dimethylcetylamine, under stirring and at a temperature of 78° C, with 0.5 mol of benzyl chloride instilled during 2 or 2.5 hours, and aging of the reaction mixture during another 3 or 4 hours [4]. The yield of the end product, dimethyl cetyl benzyl ammonium chloride, was only 92%. Moreover, a high content of tertiary amines with the alkyl radicals containing 16 and more carbon atoms causes a high toxicity of the end product.

There is a known method for producing a alkyldimethyl benzyl ammonium chloride solution by interaction of dialkyl methylamine and/or alkyldimethylamine with benzyl chloride in a solvent, alkylene glycol at a temperature of 95-100° C followed by aging of the reaction mixture during 5 hours [5]. The method is deficient in that the process time is long, and the resultant solution of a quaternary ammonium compound is weak.

There is a known method for producing alkyldimethyl benzyl ammonium chloride [6] by interaction of 603 g of tertiary alkylamine featuring a constant composition, 40% of alkyl radicals containing 12 carbon atoms, 50% of alkyl radicals containing 14 carbon atoms and 10% of alkyl radicals containing 16 carbon atoms, with 327 g of benzyl chloride in the presence of 436 g of propylene glycol at 95-100o C followed by aging during 5 hours. After that, the obtained solution was replenished with 419 g of water and additionally heated during one hour. Upon the expiration of 6 hours of the overall time of the reaction the entire reaction mass was cooled to the ambient temperature. The end product contained 50.16% of quaternary ammonium compound. Said patent also adduces other examples of interaction of tertiary amines featuring a constant composition as above with benzyl chloride in the presence of different solvents, in particular, alkyl glycols and water. As a result, a wide variety of alkyldimethyl benzyl ammonium chloride solutions was obtained with the base material concentration between 50 and 80%. The prototype method is deficient in that the end product purity is low, and the content of alkyl radicals with 16 and more carbon atoms is high both in the feed mixture of tertiary amines, and in the end product, as well as said end product requires further treatment. A process for producing alkyldimethyl benzyl ammonium chloride products used as disinfectants by reacting dodecyldimethylamine or tetradecyldimethylamine with benzyl chloride is disclosed in revue roumaine de chimie, vol. 46, no. 1, 2001, pages 49-53.

At the present time, there are fairly many preparations featuring antiseptic and simultaneously detergent properties and containing alkyldimethyl benzyl ammonium chloride consisting essentially of two fractions of tertiary amines with the alkyl radicals containing 12 and 14 carbon atoms mixed in different percentage ratios, e.g. [7]. However, isolation of each adequately pure tertiary amine with the view of its subsequent mixing presents a formidable challenge requiring numerous process steps to be executed for any one of them and, after all, a great power input. This also adds value to the end product making such household goods practically unpurchasable for the absolute majority of consumers. Besides, the existing treatment methods forbid isolation of a single tertiary amine, so not pure tertiary amines come for mixing, but a blend thereof containing larger or smaller amount of impurities, which results in the above disadvantages of the end product.

### Disclosure of the Invention.

It is an object of the invention to produce alkyldimethyl benzyl ammonium chloride consisting essentially of dodecyl dimethylbenzyl ammonium chloride and tetradecyl dimethylbenzyl ammonium chloride, to improve its purity, to reduce toxicity, as well as to simplify the process, to save energy and, as a consequence, to reduce its costs, while preserving its high efficiency as a sanitizer.

This object is accomplished by a method for producing quaternary ammonium compound consisting essentially of dodecyl dimethylbenzyl ammonium chloride and tetradecyl dimethylbenzyl ammonium chloride by a reaction of a mixture of appropriate tertiary amines with benzyl chloride under stirring and at an elevated temperature, the use is made of a mixture of commercial tertiary amines or a mixture of straight-run tertiary amines refined by rectification, and the reaction with benzyl chloride is conducted under a mass ratio of the specified mixture and benzyl chloride of 1.57 to 1.81.

Commercial tertiary amines are betain intermediates. As straight-run tertiary amines, they contain a large quantity of toxic impurities of heavy fractions of tertiary amines. Therefore, they dissatisfy sanitary and medical requirements and to date neither was used in production of detergent and disinfectant household chemical goods. An additional advantage of the claimed invention is a possibility of recycling toxic products, the mixture of commercial tertiary amines and the mixture of straight-run tertiary amines, and producing a useful cheap and effective product.

With the mass ratio of the tertiary amines-benzyl chloride mixture below 1.57, the benzyl chloride, a powerful cancerogene, content of the end product increases, which, naturally, enhances its toxicity. With the mass ratio exceeding 1.81, the end product features a higher content of unreacted tertiary amines, which also impairs the end product purity and quality.

It is preferred that benzyl chloride is refined by distillation at the atmospheric pressure and a temperature of 177-178° C is used. Such treatment makes it possible to separate an azeotropic benzyl chloride-benzaldehyde mixture, with the latter formed as a result of benzyl chloride aging. The choice of a narrow range of distillation temperatures can be accounted for the following.

The boiling point of azeotropic benzyl chloride-benzaldehyde liquid is 177.9° C. The boiling point of pure benzyl chloride is 179.3° C, barely 1.4° C over the boiling point of the azeotropic mixture. Experimentally it was found that in the temperature interval of 177-178° C, first separates the azeotropic liquid of benzyl chloride-benzaldehyde, a white nebulous liquid, and then occurs separation of a clear colorless liquid with a characteristic acrid odor, benzyl chloride. The yield of refined benzyl chloride is 55-63 mass %.

The results of the chromatography-mass spectrometry of refined benzyl chloride are presented in Table 1. The analysis was conducted using a Hewlett Packard HP-6890 chromatograph equipped with a HP-5973 mass-spectrometer detector.

### Results of the XMC Analysis of Refined Benzyl Chloride

**Table 1**

| No. | Compound | Yield time, min | Quantity, mass % |
|---|---|---|---|
| 1 | Benzyl chloride | 2.83 | 98.5 |
| 2 | Meta-chlorotoluene | 1.99 | 0.2 |
| 3 | Ortho-chlorotoluene | 2.08 | 0.1 |
| 4 | Benzaldehyde | 2.54 | 0.6 |
| 5 | Para-chlorobenzyl chloride | 3.61 | 0.6 |

As evidenced by the results presented in Table 1, the base material content of refined benzyl chloride is 98.5 mass %, while the content of each impurity does not exceed a few basis points with a total of 1.5 mass %, max. Hence, the content of foreign matter introduced by benzyl chloride into the end product, alkyldimethyl benzyl ammonium chloride, according to the reaction (1), does not exceed 0.5 mass percent in gel production, and still less in production of aqueous and non-aqueous solutions.

### Description of the Drawing.

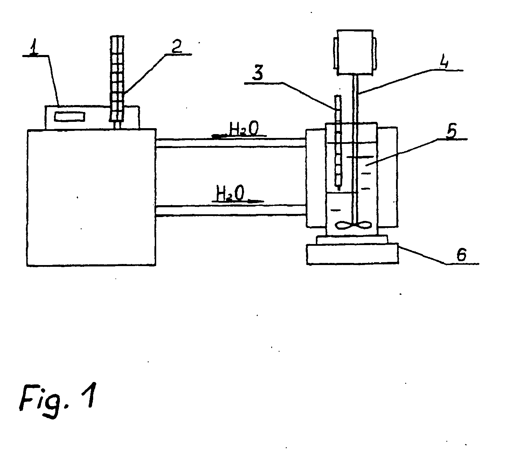

Alkyldimethyl benzyl ammonium chloride was synthesized in an installation schematically shown in Fig. 1, where 1 is a ultrathermostat, 2, 3 are temperature gages, 4 is a agitator, 5 is a thermostatted reactor with a support 6.

### Specific Embodiments

### Embodiment 1. Production of Alkyldimethyl Benzyl Ammonium Chlorides in the Form of Gel.

The thermostatted reactor 1 with a heated jacket is filled with 120 ml of a rectified commercial tertiary amines mixture consisting essentially of dodecyl dimethylamine and tetradecyl dimethylamine. The mixture is heated to 40° C under continual stirring, after which benzyl chloride is added into the reactor in small portions, while the heat is gradually increased. As the temperature rises, the solution in the reactor grows turbid, and as the temperature reaches 74° C, the turbid white solution clarifies, but tinges a kind of yellow color and gelates resembling an oily liquid. As the temperature reaches 80° C, the mixture breaks down and a white milky layer appears on its surface, which disappears when a small quantity of benzyl chloride is added. The mass ratio of the tertiary amines mixture to benzyl chloride in this embodiment is 1.57:1.

The mixture is aged in the reactor at a temperature of 80-90° C with the agitator operating at the rate of 200 rpm during 2.5 hours. Then the mixture is cooled to a room temperature. The cooling mixture rapidly gelates and congeals into a solid mass of buttercup yellow color with noticeable distinct globular inclusions.

The received alkyldimethyl benzyl ammonium chloride consists essentially of dodecyl dimethylbenzyl ammonium chloride and tetradecyl dimethylbenzyl ammonium chloride, and alkyl radicals containing more than 14 carbon atoms were not found.

### Results of Impurities Content Analysis of Alkyldimethyl Benzyl Ammonium Chloride Gel

**Table 2**

| No. | Compound | Yield time, min | Content, mass % |
|---|---|---|---|
| 1 | Alkyldimethyl benzyl ammonium chloride | | 94.89 |
| 2 | Benzyl chloride | 2.71 | 4.82 |
| 3 | Chlordecane | 5.61 | 0.06 |
| 4 | Dimethyl dodecanamine | 5.71 | 0.29 |
| 5 | Methylbenzyl dodecanamine | 9.06 | 0.04 |

As evidenced by data presented in Table 2, the derived product contains 94.89 mass percent of the base material, and the principal impurity is benzyl chloride, 4.82 mass %. This is due to the fact that benzyl chloride was taken for synthesis abundantly, and still it had no material effect on the product toxicity in general. The resultant gel features the same bactericidal effect as commercial disinfectants. Essentially, the gel can be used as a sanitizer immediately after synthesis, without any afterpurification.

### Embodiment 2

The implementation as in Embodiment 1, with the exception that the mass ratio of the tertiary amines-benzyl chloride mixture was 1.81:1. The derived alkyldimethyl benzyl ammonium chloride consists essentially of 9.9 mass percent of dodecyl dimethylbenzyl ammonium chloride and 86.8 mass percent of tetradecyl dimethylbenzyl ammonium chloride. The total amount of quaternary ammonium compounds was 96.7 mass %, and alkyl radicals containing more than 14 carbon atoms were not found. Impurities: unreacted tertiary amines - 1.1 mass %, chloroalkanes - 1.8 mass %, unreacted benzyl chloride - 0.2 mass %, other impurities - 0.2 mass %.

The toxicity of the derived product and disinfectant properties were at the level of the product according to Embodiment 1.

Embodiments 3, 4. The implementation as in Embodiments 1 and 2, with the exception that a mixture of straight-run tertiary amines was used. The results are similar to those obtained in Embodiments 1 and 2.

Embodiment 5. The implementation as in Embodiment 2, with the exception that the reactor was additionally filled with water as a thinner. The derived product was a 50-percent solution of alkyldimethyl benzyl ammonium chloride consisting essentially of dodecyl dimethylbenzyl ammonium chloride and tetradecyl dimethylbenzyl ammonium chloride.

Table 3 presents the results of the impurities content analysis of the product obtained according to Embodiment 5 (Specimen No. 2), and of the present-day commercial 50-percent aqueous solution (Specimen No. 1).

The data presented in Table 3 show that the aqueous dimethylbenzyl ammonium chloride solution obtained according to Embodiment 5 contains approximately 1.5 times less impurities than the commercial solution, and its purity is 97.23 mass %, which is higher.

The comparative results of the impurities content analysis of the commercial product and the product derived according to the invention.

**Table 3**

| No. | Compound | Specimen No. 1, mass % | Specimen No. 2, mass % |
|---|---|---|---|
| 1 | Dimethylbenzyl ammonium chloride | 95.09 | 97.23 |
| 2 | Benzyl chloride | 0.1 | 0.2 |
| 3 | Dodecene | 0.02 | 0 |
| 4 | Chlordecane | 0.2 | 0 |
| 5 | Chlordodecane | 0 | 0.01 |
| 6 | Dimethyl dodecanamine | 0 | 2.54 |
| 7 | Chlortetradecane | 0 | 0.01 |
| 8 | Alkene | 0.07 | 0 |
| 9 | Tertiary amines | 4.52 | 0 |
| 10 | Methylbenzyl dodecanamine | 0 | 0.01 |
| Total impurities: | | 4.91 | 2.77 |

Thus, the proposed invention renders it possible to produce an effective, cheap and low-toxic sanitizer with the use of inexpensive and procurable raw materials, to save energy for its production, to vary the percentage of dimethyl dodecyl ammonium chloride and tetradecyl dimethylbenzyl ammonium chloride as required, as well as to use the synthesized product without further treatment.

List of Reference.
1. Surfactants: Synthesis, Process, Applications, Development Trends, Collection of Scientific Papers, NIITEKhIM, M, 1988, p. 88
2. Report of the Crimean Medical Institute "Development and Scientific Substantiation of Maximum Allowable Concentrations of C12 - C14 Alkyldimethyl Benzyl Ammonium Chlorides", Simferopol, 1987
3. USSR Certificate of Authorship No. 355156, cl. C 07 C 21/62, 1972
4. USSR Certificate of Authorship No. 653251, cl. C 07 C 209/12, 1979
5. US Patent No. 5414124, cl. C 07 C 209/12, 1995
6. RF Patent No. 2123491, cl. C 07 C 209/12, 1998
7. RF Patent No. 2124557, cl. C 11 D 1/62, 1999

## Claims

1. A method for producing alkyldimethyl benzyl ammonium chlorides by interaction of an alkyldimethylamine mixture with benzyl chloride under stirring and at an elevated temperature **characterized in that** the base mixture of alkyldimethylamine containing dodecyl dimethylamine and tetradecyl dimethylamine - which is a mixture of commercial tertiary amines or a mixture of straight-run tertiary amines - is refined by rectification wherein the reaction with benzyl chloride is conducted under a mass ratio of the said mixture and benzyl chloride of (1.57-1.81):1.

2. A method according to claim 1, **characterized in that** the benzyl chloride is refined by distillation at the atmospheric pressure and a temperature of 177-178° C.

## Patentansprüche

1. Verfahren zur Herstellung von Alkyldimethylbenzylammoniumchloriden durch Wechselwirkung eines Alkyldimethylamin-Gemisches mit Benzylchlorid unter Rühren und bei erhöhter Temperatur, **dadurch gekennzeichnet, dass** das Dodecyldimethylamin und Tetradecyldimethylamin enthaltende Alkyldimethylamin-Grundgemisch - bei dem es sich um ein Gemisch handelsüblicher tertiärer Amine oder ein Gemisch technischer tertiärer Amine handelt - durch Rektifikation gereinigt wird, wobei die Reaktion mit Benzylchlorid mit einem Massenverhältnis des Gemisches zu Benzylchlorid von (1,57-1,81) : 1 erfolgt.

2. Verfahren nach Anspruch 1, **dadurch gekennzeichnet, dass** das Benzylchlorid durch Destillation bei atmosphärischem Druck und einer Temperatur von 177-178°C gereinigt wird.

## Revendications

1. Méthode pour produire des chlorures d'alkyldiméthylbenzylammonium par l'interaction d'un mélange d'alkyldiméthylamine avec du chlorure de benzyle sous agitation et à une température élevée **caractérisée en ce que** le mélange de base d'alkyldiméthylamine contenant de la dodécyldiméthylamine et de la trétradécyldiméthylamine - lequel est un mélange d'amines tertiaires commerciales ou un mélange d'amines tertiaires directes - est raffiné par rectification, la réaction avec le chlorure de benzyle étant effectuée avec un rapport de masse de (1.57-1.81):1 entre ledit mélange et le chlorure de benzyle.

2. Méthode selon la revendication 1, **caractérisée en ce que** le chlorure de benzyle est raffiné par distillation sous pression atmosphérique et à une température de 177-178 °C.
